# EUROPÄISCHE PATENTSCHRIFT

(11) **EP 3 659 653 B1**
(45) Veröffentlichungstag und Bekanntmachung des Hinweises auf die Patenterteilung: **09.08.2023**
(21) Anmeldenummer: 19218290.5
(22) Anmeldetag: 24.02.2015
(51) Int. Cl.: A61M 5/32, A61M 5/20

(54) **AUTOINJEKTOR MIT AUSWÄHLBAREM AUSLÖSEMODUS**
AUTOINJECTOR WITH SELECTABLE TRIGGERING MODE
AUTO-INJECTEUR AVEC MODE DE DÉCLENCHEMENT SÉLECTIONNABLE

(30) Priorität: 06.03.2014 CH 3332014
(43) Veröffentlichungstag der Anmeldung: 03.06.2020
(62) Teilanmeldung aus: 15708104.3
(73) Patentinhaber: Ypsomed AG, 3401 Burgdorf (CH)
(72) Erfinder: Hirschel, Jürg, 3007 Bern (CH); Tschirren, Markus, 3400 Burgdorf (CH); Fiechter, Marc, 3510 Konolfingen (CH)
(74) Vertreter: Meier Obertüfer, Jürg

(56) Entgegenhaltungen:
- WO-A1-2009/040602
- WO-A1-2011/005177
- AU-A1- 2010 269 142
- CH-A2- 705 992
- US-A1- 2005 101 919

## Beschreibung

Die Erfindung liegt auf dem Gebiet der Injektionsvorrichtungen zur Verabreichung eines flüssigen Produkts, insbesondere eines Medikaments. Die Erfindung betrifft eine Injektionsvorrichtung gemäss dem Oberbegriff des Anspruchs 1.

Der Begriff "Medikament" umfasst hier jede fliessfähige medizinische Formulierung, welche geeignet ist zur kontrollierten Verabreichung durch ein Mittel, wie z. B. eine Kanüle oder Hohlnadel, hindurch, beispielsweise umfassend eine Flüssigkeit, eine Lösung, ein Gel oder eine feine Suspension, welche(s) einen oder mehrere medizinische Wirkstoffe enthält. Medikament kann eine Zusammensetzung mit einem einzigen Wirkstoff oder eine vorgemischte oder co-formulierte Zusammensetzung mit mehreren Wirkstoffen aus einem einzelnen Behälter sein. Medikament umfasst Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form aber auch Polysaccharide, Vaccine, DNS oder RNS oder Oglionukleotide, Antikörper oder Teile von Antikörpern sowie geeignete Basis-, Hilfs- und Trägerstoffe. Aus dem Stand der Technik sind Injektionsvorrichtungen bekannt, die Funktionen wie automatisches Einstechen und Ausschütten aufweisen. Eine solche Injektionsvorrichtung ist aus der EP1349590 oder der US2005/0101919 oder der US20121123350 und der US2012/310156 bekannt. Diese Schriften beschreiben eine Injektionsvorrichtung welche zum Auslösen, also zum Starten der automatischen Einstech und Ausschüttbewegung der Injektionsvorrichtung eine Auslöseeinrichtung in Form eines Knopfes im proximalen Bereich der Injektionsvorrichtung aufweist. Weiter offenbaren sie eine Nadelschutzhülse welche auf eine Injektionsstelle gedrückt wird um die Nadelschutzhülse von einer distalen in eine proximale Position zu verschieben. Die Injektion kann nur dann ausgelöst werden wenn die Nadelschutzhülse auf die Injektionsstelle gedrückt ist und die Nadelschutzhülse sich in ihrer proximalen Position befindet und wenn der Knopf daraufhin betätigt wurde. Bei dieser Injektionsvorrichtung kann der Knopf nicht gedrückt werden, bevor die Nadelschutzhülse sich nicht in ihrer proximalen Position befindet.

Aufgabe der Erfindung ist es, eine verbesserte Injektionsvorrichtung anzugeben, mit der eine sichere Auslösung möglich ist.

Die Aufgabe wird gelöst durch die kennzeichnenden Merkmale des Anspruchs 1. Vorteilhafte Weiterentwicklungen ergeben sich aus den abhängigen Ansprüchen.

Die Erfindung betrifft eine Injektionsvorrichtung zur Verabreichung eines flüssigen Medikaments. Die Injektionsvorrichtung umfasst ein generell längliches Gehäuse, einen Spritzenhalter worin eine Spritze anbringbar ist und wobei die Spritze ein Medikament und einen Stopfen enthält und eine Nadel an ihrem distalen Bereich aufweist, eine Nadelschutzhülse, welche axial beweglich an dem Gehäuse angebracht ist und mit einem bestimmten Abstand aus dem distalen Ende des Gehäuses herausragt. Eine Antriebseinrichtung welche eine Kolbenstange und eine geladene Antriebsfeder aufweist und mit einer Halteeinrichtung gekoppelt und mittels einer Auslöseeinrichtung in einem gespannten Zustand gehalten wird und nach der Freigabe zunächst an der Spritze wirkt um diese nach distal zu verschieben um dann auf den Stopfen zu wirken um das Medikament aus der Spritze zu injizieren, eine Auslöseknopfeinrichtung welche einen Auslöseknopf aufweist um die Antriebseinrichtung von der Halteeinrichtung zu entkoppeln, wobei die Auslöseknopfeinrichtung so ausgestaltet ist, dass diese eine Verbindungsstelle zu der Kolbenstange aufweist, so dass eine nach distal gerichtete Bewegung der Auslöseknopfeinrichtung die Kolbenstange nach distal von einer ersten Position in eine zweite Position bewegt und über die Verbindung der Halteeinrichtung mit der Antriebseinrichtung, auch die Halteeinrichtung nach distal bewegbar ist. Wenn der Auslöseknopf gedrückt wird und von seiner proximalen Position in eine distale Position bewegt wird, kann dadurch insbesondere die Kolbenstange mit der Halteeinrichtung zusammen nach distal von der ersten Position in die zweite Position verschoben werden.

Die Auslöseeinrichtung weist ein Auslöseelement auf und ist axial beweglich im Gehäuse angebracht und mit der Nadelschutzhülse verbunden. Wenn die Nadelschutzhülse auf die Haut des Patienten aufgesetzt wird, dann wird die Nadelschutzhülse nach proximal bewegt und dadurch wird bewirkt, dass auch die Auslöseeinrichtung nach proximal bewegt wird.

Die Antriebseinrichtung ist in der Halteeinrichtung angebracht und lösbar mit der Halteeinrichtung und dem Auslöseelement gekoppelt.

Zur Auslösung der Injektionsvorrichtung muss die Antriebseinrichtung von der Halteeinrichtung entkoppelt werden, so dass die Antriebseinrichtung zunächst die Spritze zum Einstechen der Nadel nach distal bewegt um danach den Kolben zum Ausschütten des Medikaments nach distal bewegt.

Die Antriebseinrichtung ist mittels zwei Auslösemöglichkeiten auslösbar.

Der Patient kann selber auswählen welche der beiden Auslösemöglichkeiten er bevorzugt.

Eine erste Auslösemöglichkeit ist, dass der Patient zuerst den Auslöseknopf von der proximalen Position in die distale Position drückt und danach die Injektionsvorrichtung auf die Haut aufsetzt um die Nadelschutzhülse nach proximal zu bewegen und so die Injektion zu starten.

Die zweite Auslösemöglichkeit ist, dass der Patient zuerst die Injektionsvorrichtung auf die Haut aufdrückt und die Nadelschutzhülse nach proximal bewegt und so den Pen auf der Haut positioniert und bei Bereitschaft den Auslöseknopf von der proximalen Position in die distale Position drückt um die Injektion freizugeben.

In beiden Fällen wird eine sequenzielle Verschieben der Nadelschutzhülse nach proximal und eine Verschiebung des Auslöseknopfs nach distal benötigt.

Die beiden Auslösemöglichkeiten weisen folgende zwei Bewegungsabläufe auf und können in unterschiedlicher Reihenfolge erfolgen um die Injektion zu beginnen. Der erste Bewegungsablauf ist, dass mittels Drücken des Auslöseknopfs, der Auslöseknopf, die Kolbenstange in Verbindung mit der Halteeinrichtung, welche an die Kolbenstange gekoppelt ist, nach distal verschoben werden, der zweite Bewegungsablauf ist, dass die Nadelschutzhülse in Verbindung mit dem Auslöseelement nach proximal verschoben werden. Diese beiden Bewegungsabläufe bewirken, dass die Halteeinrichtung gegenüber dem Auslöseelement eine definierte Distanz verschoben wird und so die Halteeinrichtung von dem Auslöseelement freigegeben werden kann um das Antriebselement von der Halteeinrichtung zu entkoppeln und die Injektion freizugeben.

Im Folgenden wird die Erfindung anhand von Figuren beschrieben. Die dabei offenbarten Merkmale bilden je einzeln und in Kombination den Gegenstand der Erfindung vorteilhaft weiter.
- Figur 1: Explosionsdarstellung einer erfindungsgemässen Injektionsvorrichtung,
- Figuren 2 und 3: Schnittdarstellung einer erfindungsgemässen Injektionsvorrichtung mit einer aufgesetzten Kappe im Auslieferungszustand, wobei Figur 2 eine gegenüber Figur 3 um eine 90° um die Längsachse gedrehte Ansicht ist,
- Figuren 4 und 5: Schnittdarstellung einer erfindungsgemässen Injektionsvorrichtung wobei die Injektionsvorrichtung auf die Injektionsstelle aufgesetzt gezeigt wird und wobei die Figur 4 eine gegenüber der Figur 5 um eine 90° um die Längsachse gedrehte Ansicht ist,

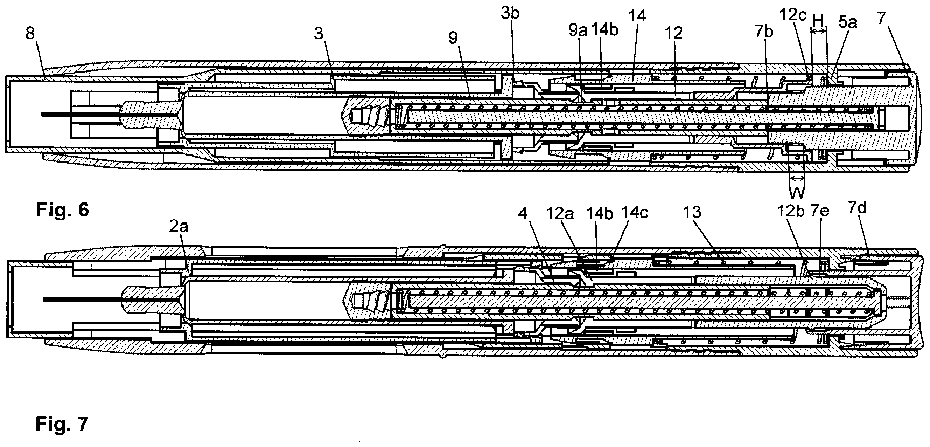

- Figuren 6 und 7: Schnittdarstellung einer erfindungsgemässen Injektionsvorrichtung wobei der Auslöseknopf von einer proximalen Position in eine distale Position gedrückt wurde und wobei die Figur 6 eine gegenüber der Figur 7 um eine 90° um die Längsachse gedrehte Ansicht ist,

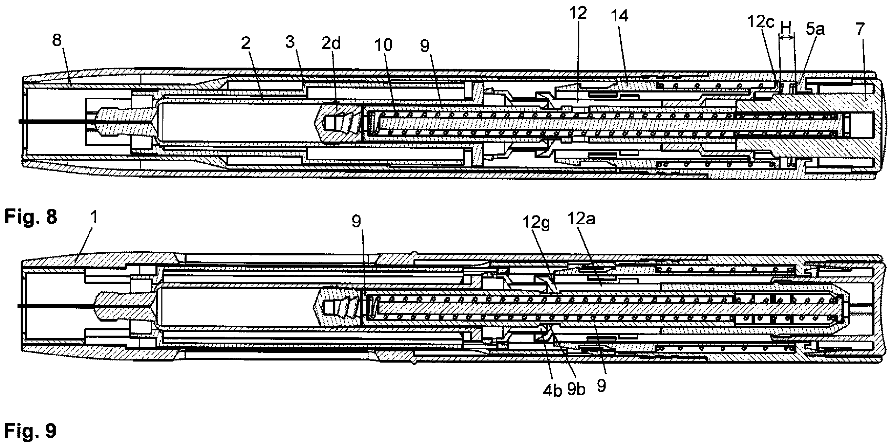

- Figuren 8 und 9: Schnittdarstellung einer erfindungsgemässen Injektionsvorrichtung wobei die Nadelschutzhülse nach proximal und der Auslöseknopf nach distal verschoben wurde und somit die Injektionsvorrichtung im ausgelösten Zustand gezeigt wird und wobei die Figur 8 eine gegenüber der Figur 9 um eine 90° um die Längsachse gedrehte Ansicht ist,

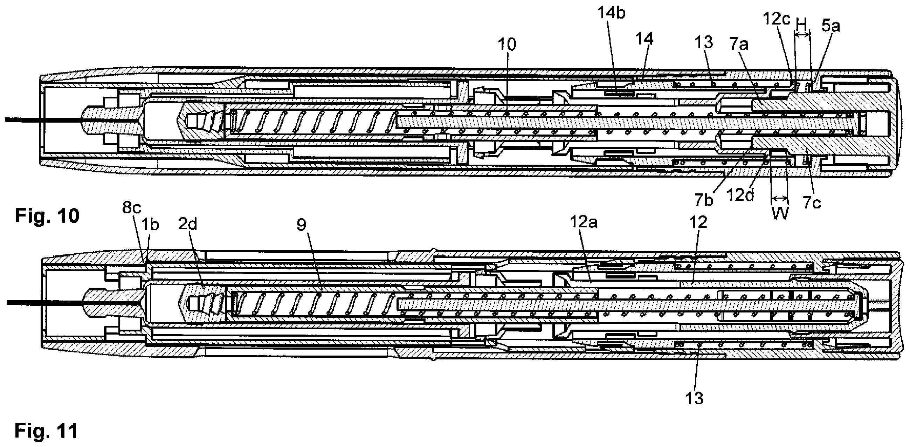

- Figuren 10 und 11: Schnittdarstellung einer erfindungsgemässen Injektionsvorrichtung wobei die Injektionsvorrichtung der Inhalt des Medikaments ausgeschüttet wurde und die Signalvorrichtung einen End-Klick verursacht hat und wobei die Figur 10 eine gegenüber der Figur 11 um eine 90° um die Längsachse gedrehte Ansicht ist,

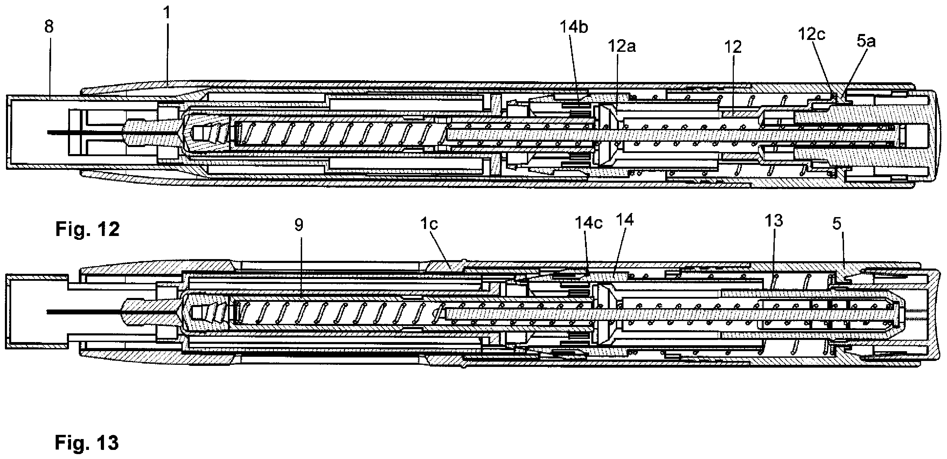

- Figuren 12 und 13: Schnittdarstellung einer erfindungsgemässen Injektionsvorrichtung wobei die Injektionsvorrichtung von der Injektionsstelle entfernt wurde und die Endverriegelung der Nadelschutzhülse stattgefunden hat und wobei die Figur 12 eine gegenüber der Figur 13 um eine 90° um die Längsachse gedrehte Ansicht ist,

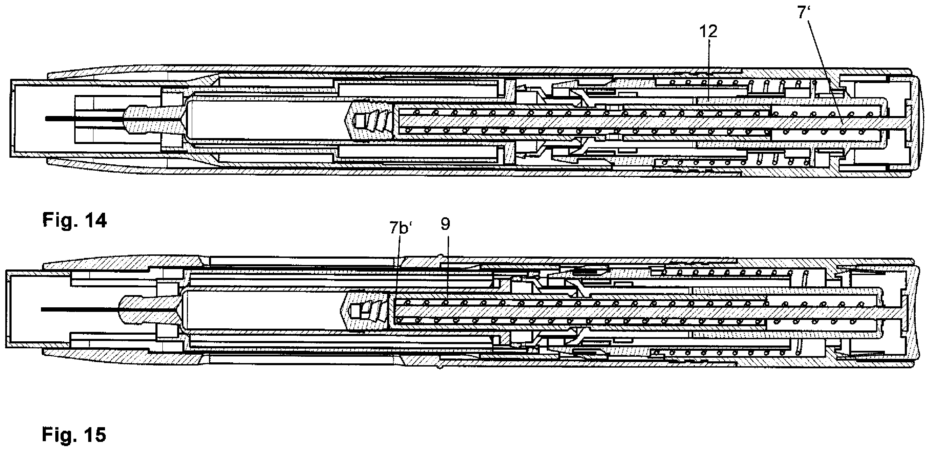

- Figuren 14 und 15: Schnittdarstellung einer erfindungsgemässen Injektionsvorrichtung mit einer anderen Ausführungsform der Auslöseknopfeinrichtung wobei die Figur 14 eine gegenüber der Figur 15 um eine 90° um die Längsachse gedrehte Ansicht ist,

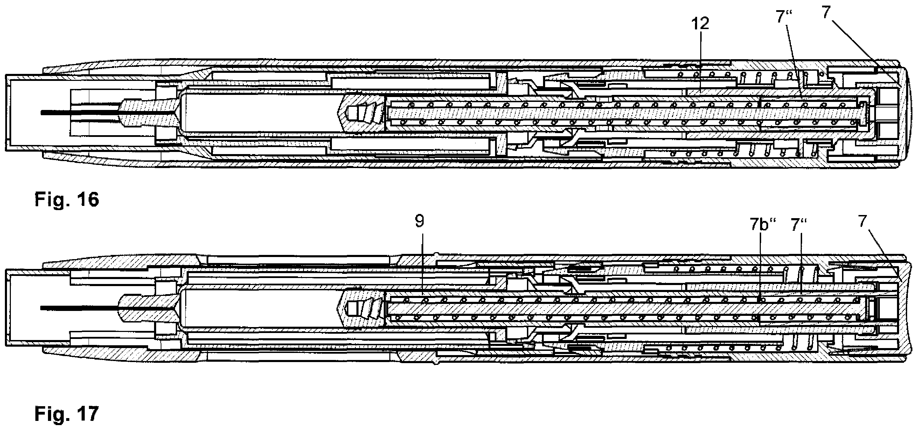

- Figuren 16 und 17: Schnittdarstellung einer weiteren erfindungsgemässen Injektionsvorrichtung mit einer anderen Ausführungsform der Auslöseknopfeinrichtung wobei die Figur 16 eine gegenüber der Figur 17 um eine 90° um die Längsachse gedrehte Ansicht ist,

### Sofern nichts anderes angegeben wird, bezeichnen gleiche Bezugszeichen die gleichen Teile

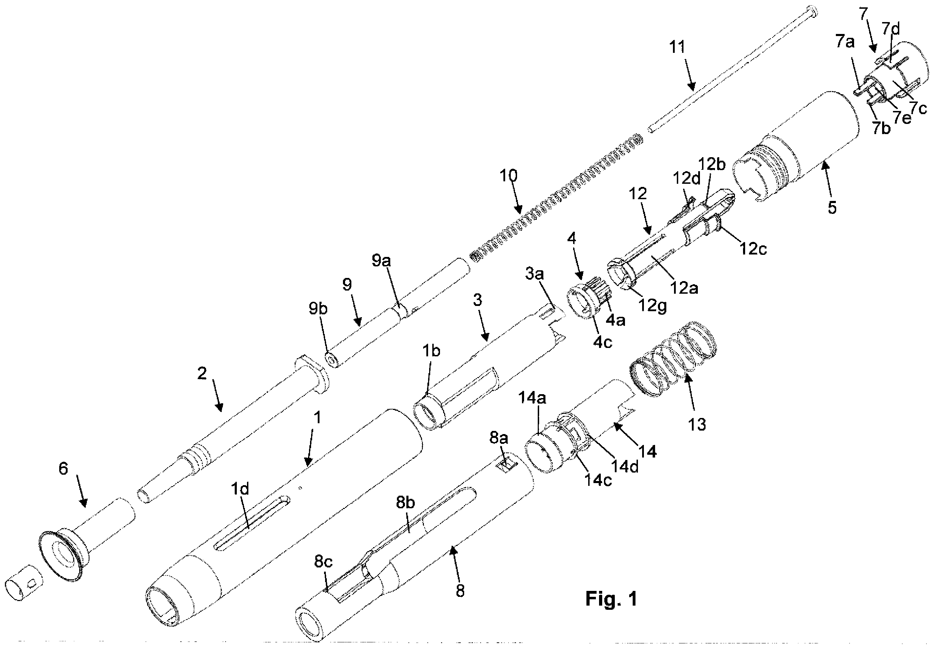

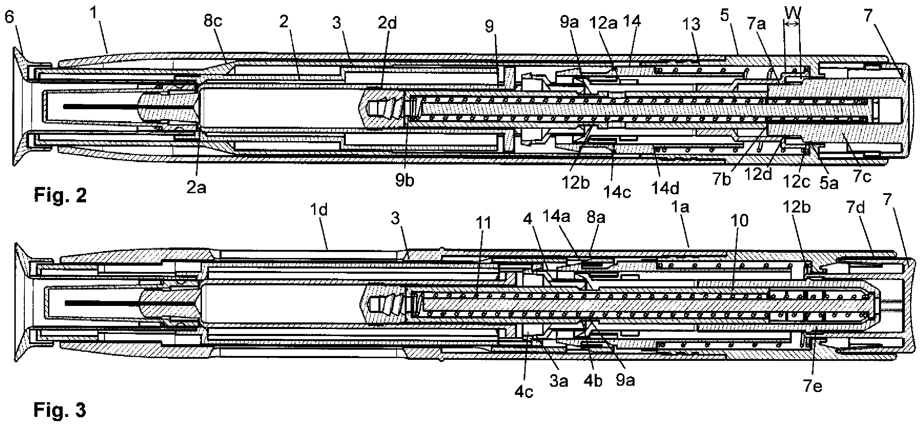

Die Injektionsvorrichtung zum Beispiel ein Autoinjektor wie in den Figuren 1, 2 und 3 gezeigt weist ein Gehäuse mit einem distalen Gehäuseteil 1 und einem proximalen Gehäuseteil 5 auf. Das distale Gehäuseteil 1 und das proximale Gehäuseteil 5 sind mit Rastverbindungen oder Gewindeverbindung miteinander verbunden.

Im distalen Gehäuseteil 1 ist ein Produktbehältnis 2 aufgenommen, an dessen distalem Ende sich eine Injektionsnadel zur Ausschüttung eines in dem Produktbehältnis 2 enthaltenen flüssigen Produkts befindet. Am proximalen Ende weist das Produktbehältnis 2 einen verschiebbaren Kolben 2d auf, dessen Bewegung relativ zum Produktbehältnis 2 und in Richtung der Injektionsnadel eine Produktausschüttung bewirkt, weshalb auch von einer Ausschüttbewegung gesprochen werden kann.

Die Injektionsvorrichtung weist ferner eine Nadelschutzhülse 8 auf, wobei die Nadelschutzhülse 8 einen distalen Bereich mit einem ersten Durchmesser und einen proximalen Bereich mit einem zweiten, grösseren Durchmesser aufweist. Aus der Figur 1 ist zumindest ein Fenster 8b an der Nadelschutzhülse 8 erkennbar wodurch auch im eingebauten Zustand die Spritze 2 sichtbar ist. An der Innenseite der Nadelschutzhülse 8 ist eine Einstechstufe 8c angebracht. Am proximalen Ende der Nadelschutzhülse 8 sind zwei Aussparungen angebracht welche nach innen gerichtete Nadelschutzhülsenanschläge 8a aufweisen. Die Nadelschutzhülse 8 ist axial verschiebbar im Gehäuse angebracht und der distale Bereich der Nadelschutzhülse 8 ragt aus dem distalen Ende des distalen Gehäuseteils 1 heraus. Die Nadelschutzhülse 8 ist mittels des Nadelschutzhülsenanschlags 8a und über Rippenanschläge 14a an einem Auslöseelement 14 fixiert, und gegen eine weitere distale Bewegung und über das Fenster 8b gegen eine Verdrehung gesichert. Dazu ragt eine Rahmenleiste des Fensters 1d radial nach innen in die Fensteröffnung 8b.

Die Vorrichtung weist ferner einen Spritzenhalter 3 auf, welcher axial beweglich in der Nadelschutzhülse 8 angebracht ist,wobei der Spritzenhalter 3 eine Schulterauflage 2a für die Fertigspritze 2 aufweist und die Fertigspritze 2 im Spritzenhalter 3 über die Schulterauflage 2a positioniert ist. Der Spritzenhalter 3 wird mittels nach proximal gerichteter Arme 3a mit nach innen weisenden Vorsprüngen 3b an die Einstechhülse 4 gekoppelt. Die Einstechhülse 4 ist ringförmig ausgebildet, sie weist im distalen Bereich einen Ringabsatz 4c auf, woran der Spritzenhalter 3 mit seinen nach proximal gerichteten Armen 3a eingehängt ist. Des Weiteren weist die Einstechhülse 4 nach proximal gerichtete flexible Arme 4a mit nach innen gerichteten Haken 4b auf, welche wiederum in der Nut 9a der Kolbenstange 9 eingeschnappt sind.

Ferner weist die Vorrichtung das Auslöseelement 14 auf, welches axial beweglich im Gehäuse angebracht und mit der Nadelschutzhülse 8 verbunden ist (wie bereits oben beschrieben). Das Auslöseelement 14 ist hülsenförmig ausgebildet und ist am distalen Ende konusförmig und weist den Rippenanschlag 14a am distalen Aussenumfang auf. Etwas beabstandet von dem Rippenanschlag 14a ist ein erster Ring 14c am Aussenumfang angebracht ein zweiter Ring 14d in einem weiteren Abstand von dem Rippenanschlag 14a angebracht. An der Innenseite weist das Auslöseelement 14 Sperrmittel 14b auf.

Die Injektionsvorrichtung weist ferner eine Halteeinrichtung 12 auf welche axial beweglich im Auslöseelement 14 angebracht ist. Die Halteeinrichtung 12 weist Federarme 12a auf, welche an ihrem distalen Bereich nach innen ragende Abragungen 12b aufweisen. Ferner weist die Halteeinrichtung 12 nach proximal gerichtete Flügel auf welche im proximalen Bereich eine Nadelschutzfederauflage 12c und etwas distal davon und an der Innenseite der Flügel angebracht, eine Abstufung 12d aufweisen.

Die Injektionsvorrichtung weist ebenfalls eine Antriebseinrichtung auf, welche axial in der Halteeinrichtung 12 angebracht ist und welche lösbar mit der Halteeinrichtung 12 und dem Auslöseelement 14 verbunden ist. Die Antriebseinrichtung wird über die Halteeinrichtung 12 und über das Auslöseelement 14 gesteuert (wird nachfolgend genauer beschrieben). Die Antriebseinrichtung ist ausgebildet um eine Antriebsenergie zu speichern und um den Spritzenhalter 3 in einem ersten Schritt nach distal zu bewegen und danach den Inhalt der Spritze 2 auszuschütten und nach Beendigung der Ausschüttung die Halteeinrichtung 12 in die proximale Richtung zu bewegen um ein akustisches und/oder taktiles Signal zu erzeugen und dem Benutzer das Ende der Injektion zu signalisieren.

Die Antriebseinrichtung weist eine Kolbenstange 9 auf welche auf den Stopfen 2d wirkt und eine Antriebsfeder 10 welche aufgeladen ist um die Kolbenstange 9 zu bewegen und in einem ersten Schritt den Spritzenhalter 3 nach distal zu treiben um die Nadel einzustechen und in einem weiteren Schritt den Stopfen 2d für die Ausschüttung des Medikaments nach distal zu bewegen. Die Kolbenstange 9 ist axial beweglich in der Halteeinrichtung 12 angebracht und die Antriebsfeder 10 ist in der Kolbenstange 9 zwischen einer distalen Wand 9b der Kolbenstange 9 und einem proximalen Ende der Halteeinrichtung 12 angebracht. Des Weiteren befindet sich ein Führungsstift 11 innerhalb der Antriebsfeder 10.

Die Kolbenstange 9 weist eine an ihrem Umfangsmantel umlaufende Nut 9a auf. Die Nut ist so ausgebildet, dass die Abragungen 12g der Federarme 12a der Halteeinrichtung 12 und die Haken 4b der flexiblen Arme 4a der Einstechhülse 4 in die Nut 9a eingreifen können.

Des Weiteren weist die Injektionseinrichtung eine Nadelschutzfeder 13 auf welche distal an dem zweiten Ring 14d des Auslöseelements 14 anliegt und proximal an der Halteeinrichtung 12 an der Nadelschutzfederauflage 12c ansteht.

Ferner weist die Einrichtung einen Auslöseknopf 7 auf. Der Auslöseknopf wird mittels Federarmen 7d im proximalen Bereich und mittels einer umlaufenden Nut 7e zu einem Ringschnapper 12b der Halteeinrichtung 12 in seiner proximalen Position gehalten. Der Auslöseknopf 7 weist einen Knopfmantel 7c auf und im distalen Bereich des Knopfmantels 7c ist an der Innenseite die umlaufende Nut 7e angebracht in die die Rippenschnapper 12b eingreifen können. Des Weiteren sind nach distal verlaufende Verlängerungsarme 7a, welche im distalen Bereich eine Auflagefläche 7b aufweisen, am Auslöseknopf 7 ausgebildet.

Im zusammengebauten Zustand sind die beiden Gehäuseteile fest verbunden. Im nicht gebrauchten Zustand wird die Kolbenstange 9 mit der Antriebsfeder 10 im gespannten Zustand gehalten. Mittels den nach innen ragenden Federarmen 12a der Halteeinrichtung 12 welche in die umlaufende Nut 9a der Kolbenstange 9 formschlüssig lösbar eingreifen und von der Auslösehülse 14 welche die Federarme 12a umgeben und so das Öffnen der Arme 12a verhindern, wird die Antriebseinrichtung im gespannten Zustand gehalten. Des Weiteren greift auch die Haltehülse 4 mittels den Haken 4b der flexiblen Arme 4a in die umlaufende Nut 9a der Kolbenstange 9 hinein. Die Spritze 2, der Spritzenhalter 3 und die Nadelschutzhülse 8 sind im distalen Gehäuseteil 1 angebracht, so dass ein Teil der Nadelschutzhülse 8 aus dem distalen Ende des distalen Gehäuseteils 1 hinausragt. Nach proximal gerichtete Arme 3a mit nach innen weisenden Vorsprüngen 3b an dem Spritzenhalter 3 greifen in den umlaufenden Ringabsatz 4c an der Einstechhülse 4. Gleichzeitig liegt der proximale Bereich der Nadelschutzhülse 8 vor dem ersten Ring 14c der Auslösehülse 14 an, wodurch proximale Bewegungen der Nadelschutzhülse 8 an die Auslösehülse 14 und distale Bewegungen der Auslösehülse 14 an die Nadelschutzhülse 8 weitergegeben werden. Das Auslöseelement 14 steht mit dem distalen Anschlag der Sperrmittel 14b an dem distalen Bereich der Federarme 12a an. Die Verlängerungsarme 7a des Auslöseknopfs 7 liegen mit der distalen Auflagefläche 7b an dem proximalen Bereich der Kolbenstange 9 an, so dass das Ende Bereich des Knopfmantels 7c einen definierten Abstand W zu der Abstufung 12d der Halteeinrichtung 12 aufweist. Die Federarme 7d des Auslöseknopfs 7 sind an einer geneigten Fläche im Inneren des proximalen Gehäuseteils 5 angelegt und bilden für eine Rückstellung des Auslöseknopfs 7 eine Getriebefläche mit dem Gehäuse.

Das Auslöseelement 14 und der Auslöseknopf 7 sind so angeordnet, dass sie unabhängig davon in welcher Sequenz sie betätigt werden miteinander agieren können und das Injektionsgerät auslösen können.

### Figuren 4 und 5:

Das Auslöseelement 14 wird als Erstes betätigt und der Auslöseknopf 7 wird als Zweites betätigt.

Wenn zunächst der distale Bereich der Nadelschutzhülse 8 gegen die Kraft der Nadelschutzfeder 13 nach proximal ins Gehäuse verschoben wird, kommt das proximale Ende der Nadelschutzhülse 8 mit dem ersten Ring 14c des Auslöseelements 14 in Kontakt und dessen Bewegung bewegt auch das Auslöseelement 14 nach proximal und die Abragungen 12g der flexiblen Federarme 12a der Halteeinrichtung 12 gelangen an das distale Ende des Auslöseelements 14. Sollte der Benutzer vor dem Drücken des Auslöseknopfes 7 die Injektionsvorrichtung von der Injektionsstelle entfernen und somit die Auslösung des Injektionsgeräts abbrechen, bewegt die geladene Nadelschutzfeder 13 das Auslöseelement 14 und somit auch die Nadelschutzhülse 8 zurück in ihre Anfangsposition.

### Figuren 6 und 7:

Der Auslöseknopf 7 wird als Erstes betätigt und das Auslöseelement 14 wird als Zweites betätigt.

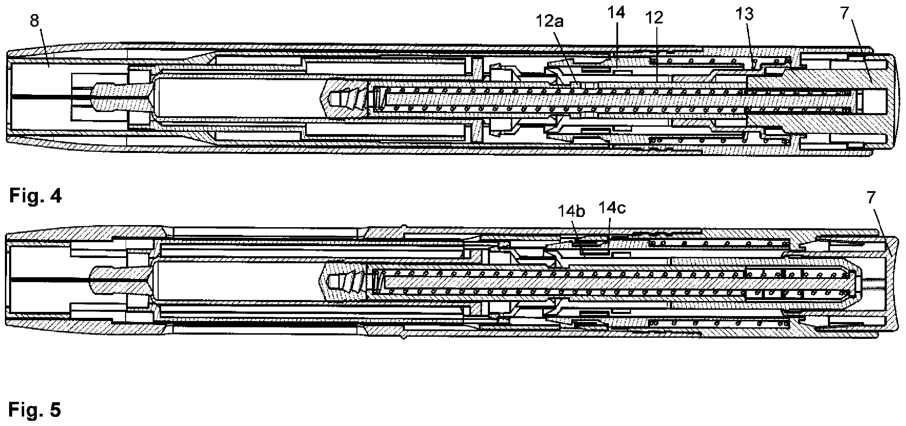

Wenn der Auslöseknopf 7 von seiner proximalen Position in eine distal Position gedrückt wird, dann kommen die Verlängerungsarme 7a mit ihren Auflageflächen 7b an den proximalen Bereich der Kolbenstange 9 auf und somit wird auch die Kolbenstange 9 und ebenso die Halteeinrichtung 12 welche an die Kolbenstange gekoppelt ist, von einer ersten Position in eine zweite Position mit nach distal verschoben. Wobei wie auch schon bei Fig. 4 und 5 beschrieben, die flexiblen Federarme 12a der Halteeinrichtung 12 an das distale Ende des Auslöseelements 14 gelangen. Ebenso wird der proximale Bereich der Nadelschutzfederauflagefläche 12c um einen Knopfhubweg H von dem Zwischenboden 5a nach distal bewegt. Durch die distale Verschiebung des Auslöseknopfs 7 und der gleichzeitigen Verschiebung bzw. Mitnahme der Halteeinrichtung 12 mittels der Kolbenstange 9 aufgrund des Eingriffs der Federarme 12a in die Nut 9a bleibt der Abstand H bei gedrücktem Auslöseknopf 7 zwischen der Abstufung 12 d und dem distalen Bereich des Knopfmantels 7c bestehen. Ferner werden die flexiblen Arme 7d des Auslöseknopfs 7 an der geneigten Fläche des Gehäuses radial nach innen gebogen und gespannt. Des Weiteren wird während der distalen Bewegung der Halteeinrichtung 12 Druck auf die Nadelschutzfeder 13, welche zwischen der Nadelschutzfederauflagefläche 12c und dem Auslöseelement 14 gespannt ist ausgeübt, wodurch das Auslöseelement 14 und über den ersten Ring 14c auch die Nadelschutzhülse 8 nach distal bewegt werden. Das Auslöseelement 14 wird so weit nach distal bewegt, bis die Sperrmittel 14b wieder an dem distalen Bereich der Federarme 12a anstehen. Sollte auch in diesem Fall der Benutzer die Auslösung des Injektionsgeräts abbrechen und den Druck von dem Auslöseknopf 7 nehmen bevor er die Nadelschutzhülse 8 auf die Injektionsstelle presst, entspannen sich die an der geneigten Gehäusefläche gespannten Federarme 7d des Auslöseknopfs 7 und der Auslöseknopf 7 bewegt sich dadurch nach proximal, wobei sich auch die Nadelschutzfeder 13 entspannt und so auch die Halteeinrichtung 12 mit in ihre Ausgangsposition gedrückt wird. Die Halteeinrichtung 12 wird bis zu dem distalen Anschlag des Anschlagelements 14b des Auslöseelements 14 nach proximal verschoben.

### Figuren 8 und 9:

Wenn nun der Auslöseknopf 7 bevor oder nachdem die Nadelschutzhülse 8 auf die Injektionsstelle gedrückt wurde und nach proximal verschoben wurde, nach distal gedrückt wird, bewirken diese beiden Bewegungsabläufe, dass die Halteeinrichtung 12 gegenüber dem Auslöseelement 14 eine definierte Distanz verschoben wird und zwar bis der distale Bereich der Federarme 12a der Halteeinrichtung 12 aus dem distalen Bereich des Auslöseelements 14 austritt und die Federarme 12a sich dadurch radial öffnen können und die Abragungen 12g aus der umlaufenden Nut 9a der Kolbenstang 9 lösen, wobei die Kolbenstange 9 frei wird und von der gespannten Antriebsfeder 10 nach distal bewegt werden kann. Dabei greifen die distalen geneigten Aussenflächen der Federarme 12a über den distalen Rand des Auslöseelements 14 wodurch die Halteeinrichtung 12 in Position gehalten wird und somit auch die proximale Seite der Nadelschutzfederauflage 12c um den Knopfhubweg H vom Zwischenboden 5a des Gehäuses entfernt gehalten wird.

Während der distalen Bewegung der Kolbenstange 9, öffnen sich die nach proximal gerichteten flexiblen Arme 4a der Einstechhülse 4 und die nach innen gerichteten Hacken 4b können sich aus der umlaufenden Nut 9a der Kolbenstange lösen. Dies geschieht, da die flexiblen Arme 4a nicht mehr von dem distalen Bereich der Federarme 12a der Halteeinrichtung gehalten werden, da sich die Federarme 12a durch ihre radiale Bewegung nach aussen im geöffneten Zustanden befinden.

### Figuren 10 und 11:

Die Kraft der Antriebsfeder 10 treibt die Kolbenstange 9 um den Stopfen 2d der Spritze 2 nach distal zu bewegen. Mittels der Antriebskraft der Antriebsfeder 10 auf den Stopfen 2d und der Reibkraft des Stopfens 2d mit dem Spritzenkörper 2, bewegt sich zunächst die Spritze 2 mit dem Spritzenhalter 3 nach distal und die Nadel wird in die Injektionsstelle eingestochen. Der Einstechvorgang wird über die Nadelschutzhülse 8 beendet, wenn der Spritzenhalter 3 mit einem Absatz 1b an die im distalen Bereich der Nadelschutzhülse 8 angebrachte Einstechstufe 8c aufkommt. Dadurch wirkt die Kraft der Antriebsfeder 10 auf den Stopfen 2d und treibt diesen in die Spritze 2 um das Medikament auszuschütten. Nachdem das Medikament ausgeschüttet wurde und das proximale Ende der Kolbenstange 9 das distale Ende der Federarme 12a der Halteeinrichtung 12 passiert hat, können sich die Federarme 12a radial nach innen bewegen und sich von dem Auslöseelement 14 lösen. Da die Kraft der Antriebsfeder 10 proximal auf die Halteeinrichtung 12 wirkt und diese nicht mehr über das Auslöseelement 14 gehalten wird, wird die Halteeinrichtung 12 axial geführt durch das Auslöseelements 14 und nach proximal bewegt, wobei die proximale Seite der Nadelschutzfederauflage 12c um den Knopfhubweg H nach proximal bewegt wird und auf den Zwischenboden 5a des Gehäuses anschlägt. Sollte der Anwender während und kurz vor dem Ende der Injektion den Auslöseknopf 7 immer noch gedrückt halten, hat die Halteeinrichtung 12 dennoch einen verfügbaren Bewegungsweg W und kann den gewünschten Weg nach proximal ausführen. Der Abstand bzw. Bewegungsweg zwischen dem distalen Ende des Knopfmantels 7c und der Abstufung 12d der Halteeinrichtung 12 ist so bemessen, dass im gedrückten Zustand des Auslöseknopfs 7, die Halteeinrichtung 12 nicht bei ihrer Bewegung nach proximal von dem Auslöseknopf 7 gehindert wird, sondern immer noch ein Bewegungsweg bzw. Abstand W zwischen der Halteeinrichtung 12 und dem Knopfmantel 7c besteht, so dass die Halteeinrichtung 12 mit ihrer Nadelschutzfederauflage 12c auf den Zwischenboden 5a aufschlagen kann, wodurch ein akustisches und/oder taktiles Signal erzeugt wird welches dem Patienten mittteilt, dass die Injektion abgeschlossen wurde und das Injektionsgerät von der Injektionsstelle entfernt werden kann. Vorzugsweise ist der freie Bewegungsweg bzw. Abstand W grösser oder gleich dem Knopfhubweg H dimensioniert.

### Figuren 12 und 13:

Wenn die Injektionsvorrichtung von der Injektionsstelle entfernt wird, dann treibt die Nadelschutzfeder 13 das Auslöseelement 14 nach distal und das Auslöseelement 14 drückt auf die Nadelschutzhülse 8 welche auch nach distal bewegt wird und aus dem distalen Gehäuseende 1 hinausragt um die Nadel abzudecken. Des Weiteren passieren die Sperrmittel 14b des Auslöseelements 14 die Federarme 12a der Halteeinrichtung 12. Nun bilden die proximalen Enden der Sperrmittel 14b und die distalen Flächen der Federarme 12a, proximale Anschläge für das Auslöseelement 14. Somit kann das Auslöseelement 14 nicht mehr nach proximal verschoben werden und auch die Nadelschutzhülse 8 ist somit für eine erneute proximale Verschiebung gesperrt.

### Figuren 14 und 15:

Eine zweite erfindungsgemässe Injektionsvorrichtung weist die selben Injektionsvorrichtungssubsysteme wie bereits in den Figuren 1 bis 3 beschrieben auf, in der zweiten Ausführungsform weist der Auslöseknopf 7 einen Verlängerungsarm 7a' auf. Der Verlängerungsarm 7a' liegt mit seinem distalen Bereich, einer Auflagefläche 7b' am inneren distalen Boden der Kolbenstange 9 auf. Wenn der Anwender den Auslöseknopf 7 der Injektionsvorrichtung drückt und von einer proximalen Position in eine distale Position verschiebt, dann verschiebt er die Kolbenstange 9 und somit auch die Halteeinrichtung 12 von einer ersten Position in eine zweite Position nach distal. Die nachfolgenden Schritte wie Beispielsweise die Auslösung erfolgen gleich wie bereits in der vorhergehenden Ausführungsform beschrieben.

### Figuren 16 und 17:

Eine weitere erfindungsgemässe Injektionsvorrichtung weist ebenfalls die selben Injektionsvorrichtungssubsysteme wie bereits in den Figuren 1 bis 3 beschrieben auf. In dieser Ausführung weist der Auslöseknopf 7 eine an der Innenseite befestigte Verlängerungshülse 7a" auf, wobei die Verlängerungshülse 7a" mit einer Auflagefläche 7b" an dem proximalen Bereich der Kolbenstange 9 anliegt und wobei beim Auslösen der Injektionsvorrichtung bzw. beim Drücken des Auslöseknopfs 7 von einer proximalen Position in eine distale Position auch die Kolbenstange 9 mit der Halteeinrichtung 12 von einer ersten Position in eine zweite Position nach distal verschoben wird. Die weiteren Auslösungsschritte folgen wie bereits beschrieben.
- 1: distales Gehäuseteil
- 1b: Absatz
- 1d: Gehäusefenster
- 2: Produktbehältnis, Spritze, Fertigspritze
- 2a: Schulterauflage
- 2d: Kolben
- 3: Spritzenhalter
- 3a: proximal gerichtete Arme
- 3b: Vorsprünge
- 4: Einstechhülse
- 4a: flexible Arme
- 4b: Hacken
- 4c: Ringabsatz
- 5: proximales Gehäuseteil
- 5a: Zwischenboden
- 7: Auslöseknopf
- 7a, 7a', 7a": Verlängerungsarme, Verlängerungshülse, Verbindungselement
- 7b, 7b', 7b": Auflagefläche
- 7c: Knopfmantel
- 7d: Federarme
- 7e: umlaufende Nut
- 8: Nadelschutzhülse
- 8a: Nadelschutzhülsenanschläge
- 8b: Fenster
- 8c: Einstechstufe
- 9: Kolbenstange
- 9a: Nut
- 9b: distale Wand
- 10: Antriebsfeder
- 11: Führungsstift
- 12: Halteeinrichtung
- 12a: Federarm
- 12b: Ringschnapper
- 12c: Nadelschutzfederauflagefläche
- 12d: Abstufung
- 12g: Abragung
- 13: Nadelschutzfeder
- 14: Auslöseelement
- 14a: Rippenanschlag
- 14b: Sperrmittel, Anschlagelement
- 14c: erster Ring
- 14d: zweiter Ring
- H: Knopfhubweg
- W: Bewegungsweg, Abstand

## Patentansprüche

1. Injektionsvorrichtung zum Injizieren von Medikamenten, aufweisend:
- ein Gehäuse mit einem distalen (1) und einem proximalen (5) Gehäuseteil;
- eine Nadelschutzhülse (8), welche in distale Richtung aus dem distalen Gehäuseteil (1) hinausragt;
- eine Nadelschutzfeder (13), welche die Nadelschutzhülse (8) aus einer proximalen Position in eine distale Position bewegen kann;
- eine Spritzenvorrichtung, aufweisend einen axial in der Nadelschutzhülse (8) bewegbaren Spritzenhalter (3) für eine Spritze (2) mit einem Kolben (2d), einem Medikament und einer Nadel an der distalen Seite, wobei die Spritze (2) im Spritzenhalter (3) angebracht werden kann;
- eine Halteeinrichtung (12);
- eine Antriebseinrichtung (9, 10), axial im Gehäuse bewegbar, und von der Halteeinrichtung (12) entkoppelbar, wobei die Antriebseinrichtung (9, 10) gespeicherte Energie aufweist um den Spritzenhalter (3) nach distal zu bewegen und um den Kolben (2d) in der Spritze (2) zum Ausschütten des Medikaments nach distal zu befördern;
- eine Auslöseknopfeinrichtung (7, 7a, 7b), aufweisend einen betätigbaren Auslöseknopf (7) um die Antriebseinrichtung (9, 10) von der Halteeinrichtung (12) zu entkoppeln,
wobei der Auslöseknopf (7) mit der Antriebseinrichtung (9, 10) und der Halteeinrichtung (12) wirkverbunden ist, **dadurch gekennzeichnet, dass** vor dem Ende der Ausschüttung, die Halteeinrichtung (12) und der Auslöseknopf (7) im gedrückten Zustand einen freien Bewegungsweg (W) zueinander aufweisen, so dass wenn die Halteeinrichtung (12) am Ende der Ausschüttung freigegeben wird, die Halteeinrichtung (12) nach proximal um den Bewegungsweg (W) bewegbar ist.

2. Injektionsvorrichtung nach Anspruch 1, **dadurch gekennzeichnet, dass** die Antriebseinrichtung (9, 10) eine Kolbenstange (9) aufweist, welche auf den Kolben (2d) wirkt um ihn in der Spritze (2) nach distal zu befördern und eine Antriebsfeder (10) mit einer Kraft, welche dazu ausgelegt ist, mittels der Kolbenstange (9) zunächst den Spritzenhalter (3) nach distal zu bewegen um dann den Kolben (2d) für eine Ausschüttung zu bewegen.

3. Injektionsvorrichtung nach einem der vorhergehenden Ansprüche, **dadurch gekennzeichnet, dass** der Auslöseknopf (7) vor der Auslösung der Injektionsvorrichtung über ein Verbindungselement (7a, 7a', 7a") mit der Kolbenstange (9) wirkverbunden ist, so dass eine distale Bewegung des Auslöseknopfs (7) die Kolbenstange (9) in distale Richtung bewegt.

4. Injektionsvorrichtung nach Anspruch 2, **dadurch gekennzeichnet, dass** die Antriebsfeder (10) zwischen einem distalen Bereich der Kolbenstange (9) und einem proximalen Bereich der Halteeinrichtung (12) angebracht ist.

5. Injektionsvorrichtung nach Anspruch 2, **dadurch gekennzeichnet, dass** die Halteeinrichtung (12) Federarme (12a) aufweist und die Kolbenstange (9) an ihrer Aussenfläche eine Nut (9a) aufweist, in welche die Federarme (12a) lösbar formschlüssig eingreifen.

6. Injektionsvorrichtung nach Anspruch 5, **dadurch gekennzeichnet, dass** die Kolbenstange (9) gegen die Kraft der Antriebsfeder (10) mittels der nach innen ragenden Federarme (12a) der Halteeinrichtung (12) welche in die umlaufende Nut (9a) der Kolbenstange (9) eingreifen gehalten wird wobei ein Auslöseelement (14) welches die Federarme (12a) umgibt das Öffnen der Federarme (12a) verhindert.

7. Injektionsvorrichtung nach Anspruch 6, **dadurch gekennzeichnet, dass** die Nadelschutzfeder (13) sich distal an dem Auslöseelement (14) und proximal an der Halteeinrichtung (12) abstützt.

8. Injektionsvorrichtung nach Anspruch 5, **dadurch gekennzeichnet, dass** die Auslöseknopfeinrichtung (7, 7a, 7b) in ihrem distalen Bereich eine Auflagefläche (7b, 7b', 7b") aufweist womit die Kolbenstange (9) mit dem Auslöseknopf (7) wirkverbunden ist und wobei durch ein Verschieben des Auslöseknopfs (7) von einer proximalen Position in eine distale Position die Kolbenstange (9) von einer ersten Position in eine zweite Position verschiebbar ist und mittels der Federarme (12a) der Halteeinrichtung (12) welche in die Nut (9a) der Kolbenstange (12) greifen auch die Halteeinrichtung (12) nach distal mitgenommen wird.

9. Injektionsvorrichtung nach Anspruch 6, **dadurch gekennzeichnet, dass** die Nadelschutzhülse (8) und das Auslöseelement (14) so angebracht sind, dass sie gemeinsam nach proximal im Gehäuse und gegenüber der Halteeinrichtung (12) verschoben werden wenn der distale Bereich der Nadelschutzhülse (8) gegen eine Injektionsstelle gedrückt wird, und dass nach der distalen Bewegung des Auslöseknopfs (7) durch diese proximale Bewegung der Nadelschutzhülse (8) die Injektion gestartet wird.

10. Injektionsvorrichtung nach einem der vorhergehenden Ansprüche, **dadurch gekennzeichnet, dass** durch eine distale Bewegung des Auslöseknopfs (7) die Antriebseinrichtung (9, 10) distal mitverschoben wird und die Halteeinrichtung (12) von der Antriebseinrichtung (9,10) nach distal mitgenommen wird.

11. Injektionsvorrichtung nach Anspruch 5, **dadurch gekennzeichnet, dass** die Halteeinrichtung (12) so ausgelegt ist, dass sie mittels der Nadelschutzfeder (13) und/oder der Antriebsfeder (10) nach proximal bewegbar ist, sobald das Ende der Kolbenstange (9) den distalen Bereich der Federarme (12a) der Halteeinrichtung (12) passiert hat und die Federarme (12a) radial nach innen bewegt sind, wobei der proximale Bereich der Halteeinrichtung (12) an einen Bereich des proximalen Gehäuseteils (5) anschlägt, wodurch ein akustisches und/oder taktiles Signal erzeugt wird.

12. Injektionsvorrichtung nach Anspruch 6, **dadurch gekennzeichnet, dass** die Nadelschutzhülse (8) und das Auslöseelement (14) mittels der Kraft der Nadelschutzfeder (13) relativ zum Gehäuse (1, 5) und zu der Halteeinrichtung (12) nach distal bewegbar sind, sobald der distale Bereich der Nadelschutzhülse (8) von der Injektionsstelle entfernt wird.

13. Injektionsvorrichtung nach Anspruch 12, **dadurch gekennzeichnet, dass** das Auslöseelement (14) Sperrmittel (14b) aufweist, so dass nachdem die Nadelschutzhülse (8) und das Auslöseelement (14) sich nach distal bewegt haben eine erneute proximale Bewegung der Nadelschutzhülse (8) verhinderbar ist.

14. Injektionsvorrichtung nach Anspruch 7, **dadurch gekennzeichnet, dass** das Auslöseelement (14) an einer Innenseite ein Sperrmittel (14b) aufweist, welches über einen distalen Anschlag an einem distalen Bereich der Federarme (12a) ansteht, so dass durch die Betätigung des Auslöseknopfes (7) vor einer Betätigung des Auslöseelementes (14) das Auslöseelement (14) ebenfalls nach distal bewegt wird.

15. Injektionsvorrichtung nach einem der vorhergehenden Ansprüche, **dadurch gekennzeichnet, dass** die Halteeinrichtung (12) am Ende der Ausschüttung mittels einer oder der Antriebsfeder (10) nach proximal bewegt wird und der proximale Bereich der Halteeinrichtung (12) an einen Bereich des proximalen Gehäuseteils (5) anschlägt, wodurch ein akustisches und/oder taktiles Signal erzeugt wird.

## Claims

1. An injection device for injecting medicaments, comprising:
a housing with a distal portion (1) and with a proximal portion (5);
a needle protection sleeve (8) protruding in a distal direction from the distal portion (1) of the housing;
a needle protection spring (13) configured to move the needle protection sleeve (8) from a proximal position into a distal position;
a syringe device comprising a syringe holder (3), which is axially moveable in the needle protection sleeve (8), a syringe (2) comprising a piston (2d), a medicament and a needle on a distal end of the syringe, wherein the syringe (2) can be placed in the syringe holder (3) of the syringe device;
a holding device (12);
a drive device (9, 10) configured to be axially moveable in the housing and to be uncoupled from the holding device (12), wherein the drive device (9, 10) stores energy for moving the syringe holder (3) in a distal direction and for discharging the medicament by advancing the piston (2d) in the distal direction in the syringe (2); and
a release button assembly (7, 7a, 7b) comprising a release button (7) for enabling the drive device (9, 10) to be uncoupled from the holding device (12),
wherein the release button (7) is operatively connected to the drive device (9, 10) and to the holding device (12), **characterized in that** before the end of the injection, the holding device (12) and the release button (7) in a depressed position are movable relative to one another by a free movement distance (W) such that when the holding device (12) is released at the end of the injection, the holding device (12) can be moved in the proximal direction over the movement distance (W).

2. The injection device of claim 1, wherein the drive device (9, 10) comprises a piston rod and a drive spring (10), wherein the drive spring (10) is configured to move the piston rod (9) such that the drive spring (10) first moves the syringe holder (3) via the piston rod (9) in the distal direction, and then moves the piston (2d) via the piston rod for discharging the medicament.

3. The injection device of claim 1 or 2, wherein before releasing the injection device, the release button (7) is operatively connected to the piston rod (9) by a connecting element (7a, 7a', 7a") such that a distal movement of the release button (7) moves the piston rod (9) in the distal direction.

4. The injection device of claim 2, wherein the drive spring (10) is arranged between a distal area of the piston rod (9) and a proximal area of the holding device (12).

5. The injection device of claim 2, wherein the holding device (12) comprises spring arms (12a), and the piston rod (9) comprises a groove (9a) on its outer surface into which the spring arms (12a) are configured to detachably engage.

6. The injection device of claim 5, wherein the piston rod (9) is held against a force of the drive spring (10) by the spring arms (12a) of the holding device (12) being in a detachable engagement with the groove (9a) of the piston rod (9), and wherein a releasing element (14) surrounds the spring arms (12a) and prevents the spring arms (12a) from opening.

7. The injection device of claim 6, wherein the needle protection spring (13) is arranged between the releasing element (14) and the holding device (12).

8. The injection device of claim 5, wherein the release button assembly (7, 7a, 7b) comprises a supporting surface (7b, 7b', 7b") operatively coupled to the piston rod (9) such that in response to a shifting of the release button (7) from a proximal position into a distal position, the piston rod (9) shifts from a first position into a second position, and the holding device (12) is shifted in the distal direction by the spring arms (12a) of the holding device, which engage in the groove (9a) of the piston rod (9).

9. The injection device of claim 6, wherein the needle protection sleeve (8) and the releasing element (14) can be shifted together in the proximal direction with respect to the holding device (12) when the needle protection sleeve (8) is pushed against an injection site, and wherein, following movement of the release button (7) in the distal direction, said movement of the needle protection sleeve (8) starts the injection.

10. The injection device of one of the preceding claims" **characterized in that** a distal movement of the release button (7) causes the drive device (9, 10) to move distally whereby the holding device (12) is shifted by the drive device (9, 10) in the distal direction.

11. The injection device according to claim 5, wherein the holding device (12) is configured to be movable by the needle protection spring (13) and/or the drive spring (10) in the proximal direction once a proximal end of the piston rod (9) has moved in a distal direction past the spring arms (12a) of the holding device (12) such that the spring arms (12a) are moved radially inward, wherein a proximal area of the holding device (12) abuts against a proximal housing portion (5) of the housing and causes an acoustic and/or tactile signal to be generated.

12. The injection device of claim 6, wherein once the distal area of the needle protection sleeve (8) is removed from the injection site, the needle protection sleeve (8) and the releasing element (14) can be moved by the force of the needle protection spring (13) in relation to the housing (1, 5) and to the holding device (12) in the distal direction.

13. The injection device of claim 12, wherein the releasing element (14) comprises blocking means (14b), such that, after the needle protection sleeve (8) and the releasing element (14) have moved in the distal direction, another proximal movement of the needle protection sleeve (8) can be prevented.

14. The injection device of claim 7, wherein the releasing element (14) comprises a blocking means (14b) arranged at an inner surface of the releasing element and abutting a distal area of the spring arms (12a), such that the release element (14) is moved in a distal direction if the release button (7) is activated before the release element (14).

15. The injection device according to one of the preceding claims, wherein the holding device (12) is configured to be movable by the drive spring (10) in the proximal direction such that a proximal area of the holding device (12) abuts against a proximal housing portion (5) of the housing and causes an acoustic and/or tactile signal to be generated.

## Revendications

1. Dispositif d'injection servant à injecter des médicaments, présentant :
- un boîtier avec une partie de boîtier distale (1) et une partie de boîtier proximale (5),
- une douille de protection d'aiguille (8), laquelle dépasse dans une direction distale de la partie de boîtier distale (1) ;
- un ressort de protection d'aiguille (13), lequel peut déplacer la douille de protection d'aiguille (8) depuis une position proximale dans une position distale ;
- un dispositif de seringue, présentant un porte-seringue (3) pouvant être déplacé de manière axiale dans la douille de protection d'aiguille (8) pour une seringue (2) avec un piston (2d), un médicament et une aiguille au niveau du côté distal, dans lequel la seringue (2) peut être installée dans le porte-seringue (3) ;
- un système de maintien (12) ;
- un système d'entraînement (9, 10), pouvant être déplacé de manière axiale dans le boîtier, et pouvant être découplé du système de maintien (12), dans lequel le système d'entraînement (9, 10) présente de l'énergie accumulée pour déplacer vers le côté distal le porte-seringue (3) et pour transporter vers le côté distal le piston (2d) dans la seringue (2) pour déverser le médicament ;
- un système à bouton de déclenchement (7, 7a, 7b) présentant un bouton de déclenchement (7) pouvant être actionné pour découpler le système d'entraînement (9, 10) du système de maintien (12),
dans lequel le bouton de déclenchement (7) est en liaison fonctionnelle avec le système d'entraînement (9, 10) et le système de maintien (12), **caractérisé en ce qu'**avant la fin du déversement, le système de maintien (12) et le bouton de déclenchement (7) dans l'état poussé présentent un trajet de déplacement (W) libre l'un par rapport à l'autre de sorte que quand le système de maintien (12) est libéré à la fin du déversement, le système de maintien (12) peut être déplacé vers le côté proximal d'une distance égale au trajet de déplacement (W).

2. Dispositif d'injection selon la revendication 1, **caractérisé en ce que** le système d'entraînement (9, 10) présente une tige de piston (9), laquelle agit sur le piston (2d) pour le transporter dans la seringue (2) vers le côté distal, et un ressort d'entraînement (10) avec une force, laquelle est configurée pour déplacer vers le côté distal au moyen de la tige de piston (9) dans un premier temps le porte-seringue (3) pour déplacer alors le piston (2d) en vue d'un déversement.

3. Dispositif d'injection selon l'une quelconque des revendications précédentes, **caractérisé en ce que** le bouton de déclenchement (7) est en liaison fonctionnelle avec la tige de piston (9) avant le déclenchement du dispositif d'inj ection par l'intermédiaire d'un élément de liaison (7a, 7a', 7a") de sorte qu'un déplacement distal du bouton de déclenchement (7) déplace dans la direction distale la tige de piston (9).

4. Dispositif d'injection selon la revendication 2, **caractérisé en ce que** le ressort d'entraînement (10) est installé entre une zone distale de la tige de piston (9) et une zone proximale du système de maintien (12).

5. Dispositif d'injection selon la revendication 2, **caractérisé en ce que** le système de maintien (12) présente des bras de ressort (12a) et la tige de piston (9) présente au niveau de sa surface extérieure une rainure (9a), avec laquelle les bras de ressort (12a) viennent en prise par complémentarité de forme de manière amovible.

6. Dispositif d'injection selon la revendication 5, **caractérisé en ce que** la tige de piston (9) est maintenue contre la force du ressort d'entraînement (10) au moyen des bras de ressort (12a) dépassant vers l'intérieur du système de maintien (12), lesquels viennent en prise avec la rainure (9a) périphérique de la tige de piston (9), dans lequel un élément de déclenchement (14), lequel entoure les bras de ressort (12a), empêche l'ouverture des bras de ressort (12a).

7. Dispositif d'injection selon la revendication 6, **caractérisé en ce que** le ressort de protection d'aiguille (13) prend appui de manière distale au niveau de l'élément de déclenchement (14) et de manière proximale au niveau du système de maintien (12).

8. Dispositif d'injection selon la revendication 5, **caractérisé en ce que** le système à bouton de déclenchement (7, 7a, 7b) présente dans sa zone distale une surface de réception (7b, 7b', 7b"), par laquelle la tige de piston (9) est en liaison fonctionnelle avec le bouton de déclenchement (7) et dans lequel par un coulissement du bouton de déclenchement (7) depuis une position proximale dans une position distale, la tige de piston (9) peut être coulissée depuis une première position dans une deuxième position et le système de maintien (12) également est entraîné vers le côté distal au moyen des bras de ressort (12a) du système de maintien (12), lesquels viennent en prise avec la rainure (9a) de la tige de piston (12).

9. Dispositif d'injection selon la revendication 6, **caractérisé en ce que** la douille de protection d'aiguille (8) et l'élément de déclenchement (14) sont installés de telle sorte qu'ils sont coulissés conjointement vers le côté proximal dans le boîtier et par rapport au système de maintien (12) quand la zone distale de la douille de protection d'aiguille (8) est poussée contre un emplacement d'injection, et que l'injection est démarrée après le déplacement distal du bouton de déclenchement (7) par le déplacement proximal de la douille de protection d'aiguille (8).

10. Dispositif d'injection selon l'une quelconque des revendications précédentes, **caractérisé en ce que** le système d'entraînement (9, 10) est entraîné vers le côté distal par un déplacement distal du bouton de déclenchement (7) et le système de maintien (12) est entraîné par le système d'entraînement (9, 10) vers le côté distal.

11. Dispositif d'injection selon la revendication 5, **caractérisé en ce que** le système de maintien (12) est configuré de telle sorte qu'il peut être déplacé vers le côté proximal au moyen du ressort de protection d'aiguille (13) et/ou du ressort d'entraînement (10) dès que l'extrémité de la tige de piston (9) a passé la zone distale des bras de ressort (12a) du système de maintien (12) et les bras de ressort (12a) sont déplacés radialement vers l'intérieur, dans lequel la zone proximale du système de maintien (12) vient buter au niveau d'une zone de la partie de boîtier (5) proximale, ce qui permet de générer un signal acoustique et/ou tactile.

12. Dispositif d'injection selon la revendication 6, **caractérisé en ce que** la douille de protection d'aiguille (8) et l'élément de déclenchement (14) peuvent être déplacés vers le côté distal au moyen de la force du ressort de protection d'aiguille (13) par rapport au boîtier (1, 5) et au système de maintien (12) dès que la zone distale de la douille de protection d'aiguille (8) est retirée de l'emplacement d'injection.

13. Dispositif d'injection selon la revendication 12, **caractérisé en ce que** l'élément de déclenchement (14) présente des moyens de blocage (14b), de sorte qu'après que la douille de protection d'aiguille (8) et l'élément de déclenchement (14) se sont déplacés vers le côté distal, un nouveau déplacement proximal de la douille de protection d'aiguille (8) peut être empêché.

14. Dispositif d'injection selon la revendication 7, **caractérisé en ce que** l'élément de déclenchement (14) présente au niveau d'un côté intérieur un moyen de blocage (14b), lequel demeure par l'intermédiaire d'une butée distale au niveau d'une zone distale des bras de ressort (12a) de sorte que l'élément de déclenchement (14) est déplacé également vers le côté distal par l'actionnement du bouton de déclenchement (7) avant un actionnement de l'élément de déclenchement (14).

15. Dispositif d'injection selon l'une quelconque des revendications précédentes, **caractérisé en ce que** le système de maintien (12) est déplacé vers le côté proximal à la fin du déversement au moyen d'un ou du ressort d'entraînement (10), et la zone proximale du système de maintien (12) vient buter au niveau d'une zone de la partie de boîtier proximale (5), ce qui permet de générer un signal acoustique et/ou tactile.
